# EUROPÄISCHE PATENTANMELDUNG

(11) **EP 1 982 658 A1**
(43) Veröffentlichungstag der Anmeldung: **22.10.2008**
(21) Anmeldenummer: 07007677.3
(22) Anmeldetag: 16.04.2007
(51) Int. Cl.: A61B 17/11, A61F 2/06

(54) **Gefässverbinder und Kit mit Applikator für die Chirurgie**

(71) Anmelder: Corlife GbR, 30625 Hannover (DE)
(72) Erfinder: Haverich, Axel, Prof. Dr., 30177 Hannover (DE); Meyer-Kobbe, Clemens, Dr., 31157 Sarstedt (DE)
(74) Vertreter: Läufer, Martina

(57) **Zusammenfassung**

Der Gefäßverbinder für das chirurgische Verbinden zweier Gefäße und/oder Prothesen miteinander umfasst einen hülsenförmigen Körper, dessen Wandung so elastisch ausgebildet ist, dass die Kraft, die auf Ligatur oder Naht wirkt in dem Maße begrenzt wird, damit keine Druckstellen oder Nekrosen auf dem Gefäß auftreten. Er besteht aus zwei den hülsenförmigen Körper begrenzenden, im wesentlichen starren äußeren Ringen und zwischen den Ringen befindliche Stege, wobei wenigstens Bereiche einzelner Stege eine von der Geraden abweichende Form größerer Weglänge besitzen, und hat wenigstens einen ringförmigen Bereich höherer Elastizität zwischen den starren Ringen.

Der Verbinder dient dem blutdichten Verbinden wenigstens zweier natürlicher oder künstlicher Gefäße einschließlich Prothese-Anschlüssen, einer Prothese mit einem natürlichen Gefäß oder auch zweier Prothesen untereinander. Der Gefäßanschluss ist mit dem neuen mechanischen Verbinder sehr viel schneller, leichter und sicherer möglich als mit konventioneller chirurgischer zirkumferentieller Naht.

## Beschreibung

### Einleitung

Die Erfindung betrifft einen Gefäßverbinder für das chirurgische Verbinden wenigstens zweier Gefäße miteinander, wobei sowohl natürliche als auch künstliche Gefäße und Prothesen eingeschlossen werden, sowie ein Kit für die Gefäßchirurgie, das aus einem solchen Gefäßverbinder und einem Applikator mit dornförmigem Ansatz besteht. Die Erfindung betrifft daher allgemein das Gebiet chirurgischer Gefäßanschlusstechniken, zum Beispiel für das Verbinden zweier natürlicher Gefäße, eines Gefäßes mit einer Prothese, bzw. mit einem Gefäßanschlussstück dieser Prothese, oder das Verbinden zweier Prothesen untereinander.

### Stand der Technik

Die klassische Verbindung zweier Blutgefäße erfolgt durch chirurgische Naht. Verschiedene Nahttechniken wie Einzelknopfnähte, Matratzennähte und fortlaufende Nähte wurden entwickelt und später auf die Verbindung eines natürlichen Blutgefäßes mit einer Gefäßprothese übertragen. Generell wird heute bei der künstlichen Verbindung eines Blutgefäßes mit einer Gefäßprothese eine fortlaufende überwendliche Naht angewendet. Auch wenn zwei Prothesen miteinander verbunden werden, wird eine fortlaufende Naht durchgeführt.

Das Nahtmaterial besteht entweder aus geflochtenem Kunststoff oder aus sogenannten monofilen Fäden. Letztere sind in der Gefäßchirurgie zu bevorzugen, da die durch sie hervorgerufenen Stichkanäle nur sehr schmal sind und es deshalb im Anschluss an die Wiederdurchblutung des Gefäßes kaum je zu einem vermehrten Blutaustritt aus den Stichkanälen kommt.

Je nach Anwendungszweck werden nicht im Körper resorbierbare Nahtmaterialien, wie Polyamide, Polyethylenterephthalate, Polypropylenarten und andere, oder im Körper resorbierbare Materialien, wie PDS (Poly-p-diaxanon), Lactid-Copolymere, Polyglactin oder andere, verwendet.

Die zirkumferentielle, das heißt den gesamten Gefäßumfang umfassende Naht ist zeitaufwendig. Im Mittel muss der operierende Arzt hierfür zwischen 6 und 10 Minuten vorsehen. Bei einer arteriellen Gefäßnaht muss die Durchströmung des Gefäßes mit Blut unterbrochen werden, so dass es zeitweise zu einer Minderdurchblutung des von der Blutader versorgten Gewebes kommt. In der Mehrzahl der anatomischen Gegebenheiten der Gefäßchirurgie ist dies nicht kritisch. Es gibt jedoch Organsysteme, in denen die Toleranz des Gewebes gegenüber einer Minderdurchblutung nur gering ist. Dies trifft insbesondere für das Gehirn zu. Hier, und auch in anderen Hauptschlagadersegmenten, können Abklemmungsphasen, die zur Herstellung von rekonstruktiven Gefäßanastomosen in Kauf genommen werden müssen, zu einer kritischen Minderdurchblutung einzelner oder auch gleich mehrerer Organe führen.

Darüber hinaus gibt es anatomische Lokalisationen, in denen nicht alle Abschnitte einer Gefäßnahtreihe gut einsehbar oder mit herkömmlichem chirurgischen Instrumentarium erreichbar sind. Als Beispiel für eine schwer zugängliche Region sei die linke Arteria subclavia genannt, wenn - wie vielfach notwendig - über eine mediane Sternotomie operiert werden muss.

Große Schwierigkeiten mit einer klassischen Naht kann es auch geben, wenn es im Zusammenhang mit der Grunderkrankung zu einer Aufspleißung der Gefäßwand (Arteriendissektion) gekommen ist. Hier kann es vorkommen, dass die in zwei Lagen gespaltene Arterienwand jeweils für sich gestochen dem Zug der Nadel und des Fadens nicht standhält und es zu Ausrissen mit anschließender Blutung aus der Gefäßanastomose kommt.

Bereits 1900 beschrieb Payr eine Anostomisierungsmethode an Gefäßen mittels resobierbarer Kanülen aus Magnesium. Später wurden andere Materialien diskutiert, wie Silber oder Elfenbein. Demichow¹ beschrieb 1958 ein Gefäßverbindungsmittel aus Kollodium. Dabei wurde über das eine Gefäßende das Röhrchen übergestülpt, wobei das Gefäß mit der Intima nach außen umgekrempelt wurde und mit einer Ligatur fixiert wurde. Das zweite Gefäß wurde über die Intima des des ersten Gefäßes gezogen und fixiert. Diese Methode birgt die Gefahr, dass sich ungefähr nach 5 Tagen Druckstellen an den Ligaturen ausbilden, die zu Nekrosen führen. Folge der Nekrosen ist das Lösen der Gefäßverbindung.

Die Patentschriften U.S. Pat. No. 6,553,812, U.S. Pat. No. 6,440,163, U.S. Pat. No. 6,309,416, U.S. Pat. No. 6,113,612 beschreiben Gefäßverbindungsmittel und zugehörige Applikationshilfsmittel. Die Gefäßverbindungsmittel sind nietenförmige Konstrukte oder rohrförmige Gefäßverbindungsmittel, welche mit Hilfe von Krampen und Widerhaken die Gefäße fixieren. Die Autoren hatten dabei vor allem die Bypass-Operation am Herzen im Blick.

Die deutsche Patentanmeldung DE10345986.9 vom 07.10.2003 beschreibt ein starres, rohrförmiges Gefäßverbindungsmittel, welches eine Perforation für eine Naht zur Fixierung der Gefäße aufweist.

### Aufgabe

Im Vergleich mit den im Stand der Technik bekannten Gefäßverbinderkonstruktionen besteht die Aufgabe der Erfindung darin, ein Hilfsmittel und ein Verfahren zu entwickeln, mit dem zwei Gefäße (künstliche Gefäße und Prothesen eingeschlossen) schnell und unkompliziert, auch in Regionen schlechter chirurgischer Erreichbarkeit oder bei Vorliegen einer Wandschwäche, dauerhaft verbunden werden können, wobei gleichzeitig die Gefahr von Durchblutungsmängeln oder Nekrosen vermieden werden soll.

Hierfür ist es erforderlich, dass Gefäßverbinder und Ligatur so zusammenwirken, dass einerseits eine stabile Verbindung hergestellt, andererseits aber der Druck auf das natürliche mit eingebundene Gewebe des Patienten möglichst gering gehalten wird.

### Lösung

Zur Lösung dieser Aufgabe zeichnet sich der erfindungsgemäße Gefäßverbinder für das chirurgische Verbinden von Gefäßen und/oder Gefäßprothesen durch Ligatur auf einem hülsenförmigen Körper durch zwei den hülsenförmigen Körper begrenzende, im wesentlichen starre äußere Ringe und zwischen den Ringen befindliche Stege sowie durch wenigstens einen ringförmigen Bereich höherer Elastizität zwischen den starren Ringen aus, wobei wenigstens Bereiche einzelner Stege eine von der Geraden abweichende Form größerer Weglänge besitzen.

Dabei bewirkt die Maßnahme, dass wenigstens Bereiche einzelner Stege - über die Länge der Hülse betrachtet - eine von der Geraden (insbesondere der direkten Verbindung zwischen den Ringen) abweichende Form größerer Weglänge besitzen, auf konstruktive Weise eine höhere Elastizität in Radialrichtung in diesem Bereich, wie sie für die Zwecke der Erfindung gewünscht ist.

Gerade durch die erfindungsgemäßen Maßnahmen ist der hülsenförmige Körper des Gefäßverbinders so beschaffen, dass die Kraft, die auf Gefäßwand und Naht oder Ligatur wirkt begrenzt wird, so dass keine Druckstellen oder Nekrosen auftreten. Dennoch kann per Ligatur oder Naht eine blutdichte Verbindung hergestellt werden.

Der hülsenförmige Körper oder Rohrkörper ist in bevorzugter Ausführungsform im Wesentlichen zylindrisch. Der Körper hat dann die Form einer einfachen zylindrischen Hülse. Es ist jedoch nicht ausgeschlossen, dass es für bestimmte Anwendungen zweckmäßig sein kann, wenn der hülsenförmige Körper bogen- oder s-förmig ist.

Die Länge des Verbindungsmittels beträgt vorzugsweise insgesamt ca. 4 bis 16 mm. Der Durchmesser des Verbindungsmittels beträgt vorzugsweise zwischen 4 mm und 30 mm.

Für die Ligatur kann monofiles Nahtmaterial verwendet werden, vorzugsweise jedoch polyfiles Material, wie z.B. Nabelband aus Baumwolle (Durchmesser ca. 2 mm).

Die äußeren Ringe des Gefäßverbinders sind starr, d.h. bei normaler Handhabung und bestimmungsgemäßem Gebrauch in ihrer Form nicht veränderlich oder im Wesentlichen starr, was bedeuten soll, dass sie relativ zu dem wenigstens einen an dem Verbinder vorhandenen ringförmigen Bereich höherer Elastizität weniger elastisch sind.

Die Elastizität des ringförmigen Bereiches bezieht sich auf das Verhalten bei in Radialrichtung ausgeübtem Druck. Gegenüber radial ausgeübtem Druck soll der Bereich sich elastisch verhalten, d.h. in Richtung auf die Mittelachse des Verbinders nachgeben. Dieses Verhalten soll relativ elastischer sein als das der starren äußeren Ringe.

Das Maß der Elastizität richtet sich nach der chirurgischen Anwendung und ist durch die erfindungsgemäßen Merkmale so wie weiter unten anhand der Beispiele noch näher beschrieben automatisch erzielbar. Die Elastizität ergibt sich also durch das Stegdesign und ist mit dessen Hilfe einstellbar.

Zwischen den äußeren Ringen befinden sich Stege. Diese Stege können mit den äußeren Ringen und anderen als stegförmigen, z.B. platten- oder ringförmigen Bereichen verbunden sein. Vorzugsweise sind jedoch die äußeren Ringe mit von einem zum anderen Ring durchlaufenden Stegen verbunden.

Gemäß einer Ausführungsform der Erfindung befindet sich der ringförmige Bereich höherer Elastizität in der Mitte des Gefäßverbinders oder es sind mehrere ringförmige Bereiche symmetrisch zur Mitte angeordnet.

Für die ringförmigen Bereiche höherer Elastizität ist es bevorzugt, dass sämtliche diesen durchlaufenden Stege innerhalb des Bereichs verlängerte Weglängen besitzen.

Die von der Geraden abweichende Form größerer Weglänge wird erreicht, indem die Stege so ausgebildet werden, dass der einzelne Steg zwischen zwei Punkten A und B einen Weg beschreibt, der länger ist als die direkte, gerade, d.h. kürzeste Verbindung zwischen A und B. In bevorzugter Ausführung wird dies durch eine s-förmigen, z-förmigen, wellenförmigen oder meandernden Stegverlauf bewirkt. Die Vergrößerung der Weglänge der Stege gegenüber der Geraden sollte wenigstens 10 % betragen.
Die Stege können unverzweigt, verzweigt, wenigstens bereichsweise vernetzt oder wie bei einem Gewebe oder Gewirk verschränkt ausgebildet sein.

Bei einem besonders bevorzugten Ausführungsbeispiel der Erfindung umgeben zwei ringförmige Bereiche s- oder z- oder wellenförmigen oder meandernden Stegverlaufs einen mittleren Bereich im wesentlichen gerader Stegabschnitte, um so einen ringförmigen Bereich höherer Elastizität zu bilden, so dass sich insgesamt ein Bereich höherer Elastizität ergibt. Schnürt man diesen Gefäßverbinder im Bereich der geraden Stegabschnitte, d.h. ungefähr in der Mitte des elastischen Bereichs ringförmig radial ein, ergibt sich bei hinreichendem Druck ein in etwa U-förmig eingezogenes Profil. Durch die Möglichkeit, ein solches Profil auszubilden, wird gleichzeitig die Naht auf dem hülsenförmigen Körper festgelegt, da sie sich durch das Fixieren automatisch an einem Punkt relativ geringsten Durchmessers befindet.

Allgemein können daher bei der Erfindung Mittel zum Festlegen einer Ligatur über dem Verbinder dadurch gebildet werden, dass ein mittlerer ringförmiger Bereich höherer Elastizität von demgegenüber starreren, hülsenförmigen, an die äußeren Ringe angrenzenden Bereichen umgeben ist. Dies kann bei mehreren Elastischen Bereichen auch mehrfach an einem Gefäßverbinder geschehen, um beispielsweise an einem Verbinder mehrere mögliche Positionen für die Ligatur anzubieten, oder um mehr als zwei Gefäße oder Prothesen verbinden zu können. Die genannten Mittel können auch mehrfach vorgesehen sein, um verschiedene Ligaturen einzeln festzulegen, z. B. an einem verzweigten Gefäßverbinder. Hierfür ist es beispielsweise möglich, dass ein mittlerer ringförmiger Bereich relativ geringerer Elastizität von zwei ringförmigen Bereichen relativ höherer Elastizität umgeben ist. Ein verzweigter Gefäßverbinder kann vorzugsweise T- oder Y-förmig verzweigt sein.

Selbstverständlich ist es außerdem möglich, dass der hülsenförmige Körper bereits in relaxierter oder Ruheposition vom Rand zur Mitte hin im Durchmesser abnimmt, oder allgemein mehrere Positionen geringeren Durchmessers aufweist.

Allgemein ist der Wandaufbau des Gefäßverbinders so ausgebildet, dass die Wandung des hülsenförmigen Körpers an bestimmten Stellen so dünn oder ganz durchbrochen ist, dass insgesamt eine elastische Struktur entsteht. Vorzugsweise kann die gewünschte Struktur aus Rohr geschnitten werden oder aus einem Geflecht aus Drähten bestehen.

Materialstärke und Materialeigenschaften der Wandung werden so gewählt, dass eine ausreichende Stabilität zur Fixierung der Prothese und/oder der Gefäße gewährleistet ist, sich jedoch an den Gefäßen keine Druckstellen an der Ligatur bzw. Naht ausbilden, die zu Nekrosen führen können.

Gemäß einer besonders bevorzugten Ausführungsform der Erfindung sind die Stege aus dem hülsenförmigen Körper des Gefäßverbinders freigeschnitten. Die durchbrochene Ausführungsform besitzt einerseits den Vorteil, dass die dadurch gegebenen Durchlässe von Nadeln oder sonstigen chirurgischen Nähwerkzeugen zum Durchziehen eines chirurgischen Nahtmaterials durchstochen werden können.

Bei der Ligatur wird das Verbindungsmittel in die Prothese oder den anzusetzenden Gefäßstumpf eingeführt. Bei einem einfachen Verbindungsmittel mit nur einer Ligaturposition wird das Verbindungsmittel mit der darübergezogenen Prothese oder dem darübergezogenen Gefäßstumpf gemeinsam in das offene Ende des anzubindenden Gefäßes gesteckt, so dass ein dreischichtiger Aufbau entsteht (von innen nach außen: Verbindungsmittel, Prothese/Gefäß, Gefäß). Alle drei Schichten gemeinsam werden an der dafür vorgesehenen Position mit einem Faden umschlungen (oder umwickelt, ca. 1-3 Wicklungen) und dann mittels eines chirurgischen Knotens zugeknotet. Bei einem Verbindungsmittel mit wenigstens zwei Ligaturpositionen kann die erste Ligatur im Falle einer anzubindenden Prothese vor der Operation vorbereitet werden, indem das Verbindungsmittel einseitig in die Prothese gesteckt und durch Ligatur (wie oben beschrieben, jedoch mit zur 2 Schichten, nämlich Verbindungsmittel und Prothese) verbunden wird. Bei der Operation wird die vorbereitete Prothese mit dem einseitig noch freien Verbindungsmittel in den offenen Gefäßstumpf gesteckt und in gleicher Weise (2schichtig) durch Ligatur verbunden. Zwei natürliche Gefäße werden während einer Operation in entsprechender Weise verbunden.

Die durchbrochene Ausführungsform besitzt weiterhin den Vorteil, dass eine ausreichende Mikrozirkulation im Naht- bzw. Ligaturbereich und damit eine ausreichende Blut- und Sauerstoffversorgung der Gefäße über die Naht bzw. Ligatur hinaus gewährleistet ist, so dass die Nekrosegefahr hierdurch zusätzlich herabgesetzt wird.

In alternativer Ausführungsform können sich die Stege sich auf einer folienartig dünnen Hülse reliefartig erheben. Hierfür wird das Material zwischen den Stegen folienartig abgetragen, beispielsweise mit einem Laser. Wenigstens im Bereich der Fixierung bzw. Ligatur ist der Gefäßverbinder zu 50 bis 80 % der Fläche durchbrochen oder abgetragen.

Der Gefäßverbinder kann aus allen mechanisch geeigneten Materialien hergestellt werden, die für die Verwendung im menschlichen und tierischen Körper zugelassen sind und hinreichende Festigkeit haben. Viele derartige Materialien, die beispielsweise auch für orthopädische und zahnmedizinische Implantate, chirurgische Instrumente, Herzklappen und dergl. verwendet werden, sind dem Fachmann auf dem Gebiet der Chirurgie und Implantologie bekannt.

In einer bevorzugten Ausführungsform besteht der Gefäßverbinder aus Metall, vorzugsweise Titan, einer Titanlegierung oder Edelstahl.

Allgemein kommen als Materialien in Frage: Metalle, insbesondere Titan oder Edelstahl, einschließlich der besonderen, für Implantate und medizinische Instrumente verwendeten Legierungen, Karbon-Materialien, einschließlich Karbonfaser-Netzen, Weichkunststoff, wie beispielsweise Silikon, Hartkunststoff, wie beispielsweise Teflon, keramisches Material und biologisch resorbierbares Material.

Gemäß einer möglichen Ausführungsform kann der rohrförmige oder hülsenförmige Körper des Gefäßverbinders einen im wesentlichen zylindrischen Mittelteil aus einem relativ härteren Material und glatte oder trompetenförmige Ansätze aus einem relativ weicheren Material besitzen. Die Ligatur oder Naht wird dann im Bereich des härteren Materials angelegt, während die ausgeweiteten oder nur weicheren Enden des Gefäßverbinders ein Anmodellieren an die Gefäßwand/Prothesenwand ermöglichen.

Der Gefäßverbinder nach der Erfindung kann ganz oder teilweise mit einer das Anhaften von Blutbestandteilen verhindernden oder zumindest vermindernden Beschichtung und/oder Struktur versehen sein, vorzugsweise ganz oder teilweise auf der Innenseite, d.h. luminal.

Eine solche Beschichtung kann aus einem die Oberfläche glättenden oder die Gleitfähigkeit auf der Oberfläche erhöhenden Material bestehen. Für eine die Gleitfähigkeit erhöhende Beschichtung kann beispielsweise ein Polybutylat oder eine Mischung oder ein Copolymer mit Polybutylat verwendet werden. Die Beschichtung könnte auch antithrombotische Medikamente, z.B. Heparin enthalten. Ferner ist es möglich, eine Beschichtung vorzusehen, die einen Lotuseffekt auf der Oberfläche erzeugt. Die Lotusstruktur kann alternativ auch direkt an der Oberfläche des hülsenförmigen Körpers - ohne gesondert Beschichtung - ausgebildet werden. Beschichtete und strukturierte Oberflächen für medizinische Geräte sind beispielsweise aus der WO 00/07633 oder der DE 199 50 452 bekannt.

In Weiterbildung der Erfindung weist der Gefäßverbinder wenigstens an einem der äußeren Ringe einen kragenförmigen Ansatz als Anschlag für einen in den Gefäßverbinder einzuführenden dornförmigen Applikator auf.
Die Erfindung umfasst daher weiterhin ein Kit aus einem Gefäßverbinder und einem Applikator mit dornförmigem Ansatz für die chirurgische Verbindung von Gefäßen und/oder Gefäßprothesen, vorzugsweise per Ligatur. Der Gefäßverbinder wird auf den dornförmigen Ansatz gesteckt und mit diesem in Position gebracht, wie anhand der Figuren noch näher beschrieben. Der Dorn dient zum einen als ein Begrenzungsmittel, damit nicht zu fest zugeschnürt werden kann, zum anderen wird das Verbindungsmittel an der richtigen Stelle positioniert.

Im Folgenden wird die Erfindung anhand von in der Zeichnung dargestellten Beispielen näher erläutert. Die Beispiele wurden zu illustrativen Zwecken ausgewählt, eine Beschränkung der allgemeinen Möglichkeiten der Erfindung soll hierdurch nicht erfolgen. Im Einzelnen zeigen:
- Fig. 1: erstes Ausführungsbeispiel eines Gefäßverbinders in Seitenansicht;

- Fig. 2: Detailansichten für den Anschluss von Stegstrukturen an einen äußeren Ring;
- Fig. 3: Detailansichten für Strukturen mit Vergrößerung der Weglänge;
- Fig. 4: Detailansichten für Mittelbereiche/Nahtfestlegungsbereiche;
- Fig. 5: Querschnittsansicht durch eine teilweise folienartig abgetragene Wandung;
- Fig. 6: zweites Ausführungsbeispiel eines Gefäßverbinders in Seitenansicht;
- Fig. 7: Querschnittsansicht durch Applikator, Gefäßverbinder, T-förmige Gefäßprothese und anzuschießenden Gefäßstumpf in Operationssituation;
- Fig. 8: Querschnittsansicht eines alternativen Applikatorbeispiels in Anwendungsposition.

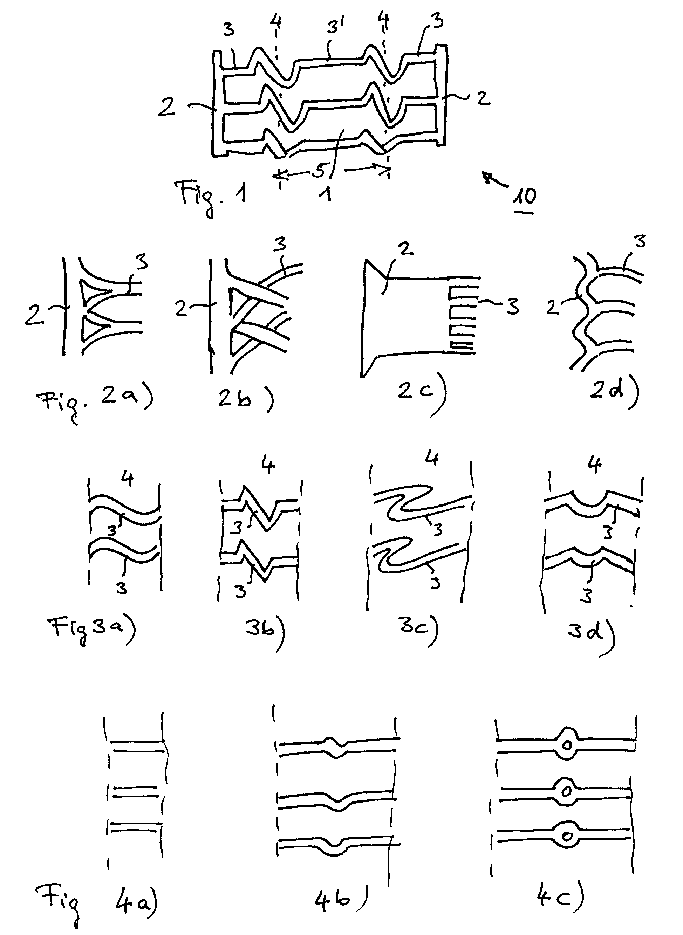

Figur 1 zeigt ein erstes Ausführungsbeispiel für einen im Ganzen mit 10 bezeichneten Gefäßverbinder aus einem hülsenförmigen Körper 1, der an seinen Rändern durch zwei im Wesentlichen starre Ringe 2 begrenzt ist. Die Ringe 2 sind durch von einem Ring zum anderen durchlaufende Stege 3 verbunden. Entlang zweier ringförmiger, hier symmetrisch zur Mitte angeordneter Bereiche weisen die Stege 3 Wellen 4 auf, die jeweils von der Geraden abweichende Formen größerer Weglänge bilden (jeweils allgemein als 4 bezeichnet). Durch Strecken dieser wellenförmigen Strukturen wird mit konstruktiven Mitteln eine gut einstellbare Elastizität erzeugt. Hierdurch erstreckt sich an diesem Gefäßverbinder 10 ein ringförmiger Bereich 5 insgesamt höherer Elastizität über beide wellenförmigen Bereiche und die dazwischen liegenden geraden Stegabschnitte 3', auf denen die Ligatur festgelegt werden kann.

Figur 2 zeigt verschiedene mögliche Strukturen für die Randbereiche eines Gefäßverbinders 10 am Übergang zwischen Ring 2 und Stegen 3. Diese Randbereiche sollen jeweils steifer als mindestens ein zwischen den Ringen liegender ringförmiger elastischer Bereich 5 sein, können aber selbst dennoch eine gewisse eigene Elastizität aufweisen.

Figur 3 zeigt verschiedene mögliche Strukturen für die von der Geraden abweichenden Formen 4 größerer Weglänge innerhalb der Stege 3. Durch Strecken dieser Strukturen bzw. Formen 4 bei Belastung wird konstruktiv Elastizität bereitgestellt. Je nach Gesamtdesign, kann der ringförmige Bereich 5 höherer Elastizität mit einem einzelnen ringförmigen Bereich verlängerter Stegweglängen (verschiedene Formen 4) zusammenfallen, oder der Bereich 5 höherer Elastizität wird durch zwei derartige Bereiche verlängerter Stegweglängen mit dazwischen liegender Ligaturzone gebildet (beispielsweise aus geraden Stegabschnitten). Ein Beispiel hierfür ist wird in Figur 1 gezeigt.
Figur 4 zeigt mögliche Strukturen für Mittelbereiche der hülsenförmigen Körper 1, die zwischen zwei Bereichen, wie in Figur 3 gezeigt, liegen können.

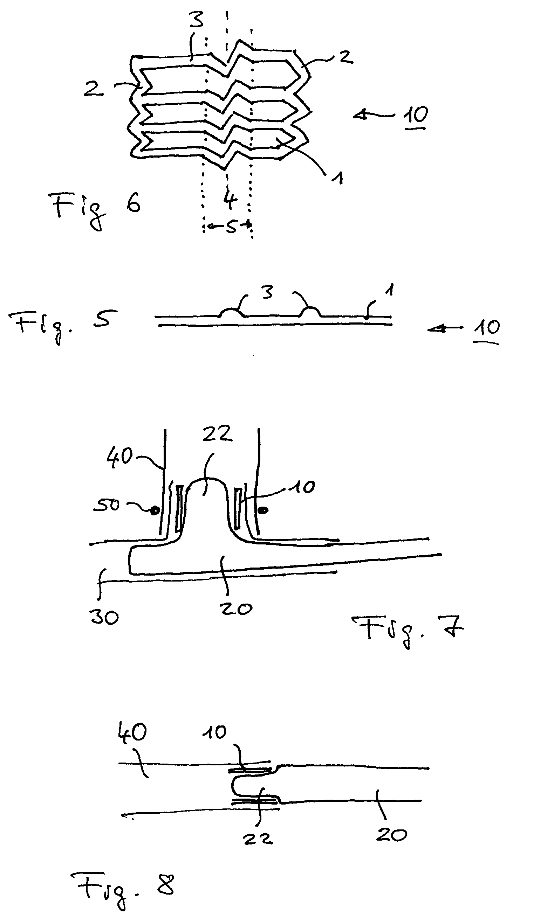

Figur 5 illustriert eine alternative Ausführungsform: gezeigt ist ein Querschnitt durch die Seitenwand des Verbinders 10. In diesem Falle erheben sich die Stege 3 nur reliefartig auf einem folienhaft dünnen hülsenförmigen Körper 1. Hierfür wird das Material zwischen den Stegen 3 beispielsweise mit einem Laser abgetragen, bis nur noch eine folienartige Schicht des ursprünglich gleichförmig dicken zylindrischen Grundkörpers übrig bleibt. Die Schicht lässt sich sehr leicht verbiegen oder verwinden, so dass eine entsprechende Elastizität wie bei dem durchbrochenen Ausführungsbeispiel erreicht werden kann.

Figur 6 zeigt ein weiteres Ausführungsbeispiel mit gezackten Ringen 2. Diese Struktur kann sich gegebenenfalls empfehlen, um sich unregelmäßigen Gefäßformen besser anzupassen. Im übrigen bezeichnen gleiche Bezugszeichen gleiche Bauteile.

Figur 7 zeigt ein Ausführungsbeispiel für einen mit einem Applikator 20 in eine Gefäßprothese 30 eingeführten Gefäßverbinder 10 in der Operationssituation. Der Gefäßverbinder 10 wird dabei zunächst auf einen Dorn 22 des Applikators aufgesetzt und durch die Prothese 30 an die Verankerungsposition geführt. Ein anzuschließendes Gefäß 40 wird über das Ende der Prothese 30 mit dem darunter liegenden Gefäßverbinder 10 gezogen. Der Gefäßverbinder behält seine feste Position durch den Applikator 20 bei. Schließlich wird der Gefäßverbinder 10 mit der Prothese 30 und dem Gefäß 40 verschnürt. Die Ligatur ist bei 50 angedeutet. Der Dorn 22 dient zum einen als ein Begrenzungsmittel, und verhindert eine zu straffe Ligatur, und hält gleichzeitig den Gefäßverbinder formschlüssig an der gewünschten Position. Gegebenenfalls kann an dem Dorn ein Anschlag vorgesehen sein, gegen den der Gefäßverbinder beim Einschieben in das Gefäß anschlägt.

Figur 8 zeigt ein alternatives Ausführungsbeispiel für einen Applikator 20, der für andere Gefäß- bzw. Prothesegeometrien vorgesehen ist in der zugehörigen Anwendungsposition (skizziert).

## Patentansprüche

1. Gefäßverbinder (10) für das chirurgische Verbinden von Gefäßen und/oder Gefäßprothesen durch Ligatur auf einem hülsenförmigen Körper (1), **gekennzeichnet durch** zwei den hülsenförmigen Körper (1) begrenzende, im wesentlichen starre äußere Ringe (2) und zwischen den Ringen befindliche Stege (3), wobei wenigstens Bereiche einzelner Stege (3) eine von der Geraden abweichende Form (4) größerer Weglänge besitzen, sowie **durch** wenigstens einen ringförmigen Bereich (5) höherer Elastizität zwischen den starren Ringen (2).

2. Gefäßverbinder nach Anspruch 1, **dadurch gekennzeichnet, dass** die äußeren Ringe (2) mit durchlaufenden Stegen (3) verbunden sind.

3. Gefäßverbinder nach Anspruch 1 oder 2, **dadurch gekennzeichnet, dass** sich der ringförmige Bereich (5) höherer Elastizität in der Mitte des Gefäßverbinders (10) befindet, oder dass mehrere ringförmige Bereiche (5) symmetrisch zur Mitte angeordnet sind.

4. Gefäßverbinder nach einem der Ansprüche 1 bis 3, **dadurch gekennzeichnet, dass** in dem ringförmigen Bereich (5) höherer Elastizität diesen durchlaufenden Stege (3) innerhalb des Bereichs verlängerte Weglängen besitzen.

5. Gefäßverbinder nach einem der Ansprüche 1 bis 4, **dadurch gekennzeichnet, dass** die von der Geraden abweichende Form (4) größerer Weglänge durch eine s-förmigen, z-förmigen, wellenförmigen oder meandernden Stegverlauf bewirkt wird.

6. Gefäßverbinder nach Anspruch 5, **dadurch gekennzeichnet, dass** zwei ringförmige Bereiche s- oder z- oder wellenförmigen oder meandernden Stegverlaufs einen mittleren Bereich im wesentlichen gerader Stegabschnitte umgeben, so dass sich insgesamt ein Bereich (5) höherer Elastizität ergibt.

7. Gefäßverbinder nach einem der Ansprüche 1 bis 6, **dadurch gekennzeichnet, dass** die Vergrößerung der Weglänge der Stege gegenüber der Geraden wenigstens 10 % beträgt.

8. Gefäßverbinder nach einem der Ansprüche 1 bis 7, **dadurch gekennzeichnet, dass** die Stege (3) unverzweigt, verzweigt, wenigstens bereichsweise vernetzt oder verschränkt ausgebildet sind.

9. Gefäßverbinder nach einem der Ansprüche 1 bis 8, **dadurch gekennzeichnet, dass** Mittel zum Festlegen einer Ligatur über dem Verbinder **dadurch** gebildet werden, dass ein mittlerer ringförmiger Bereich (5) höherer Elastizität von demgegenüber starreren, hülsenförmigen, an die äußeren Ringe angrenzenden Bereichen umgeben ist.

10. Gefäßverbinder nach einem der Ansprüche 1 bis 8, **dadurch gekennzeichnet, dass** Mittel zum Festlegen einer Ligatur über dem Verbinder **dadurch** gebildet werden, dass ein mittlerer ringförmiger Bereich (5) relativ geringerer Elastizität von zwei ringförmigen Bereichen relativ höherer Elastizität umgeben ist.

11. Gefäßverbinder nach einem der Ansprüche 1 bis 10, **dadurch gekennzeichnet, dass** die Stege (3) aus dem hülsenförmigen Körper (1) des Gefäßverbinders freigeschnitten sind.

12. Gefäßverbinder nach einem der Ansprüche 1 bis 10, **dadurch gekennzeichnet, dass** die Stege (3) sich auf einer folienartig dünnen Hülse reliefartig erheben.

13. Gefäßverbinder nach einem der Ansprüche 1 bis 12, **dadurch gekennzeichnet, dass** er aus Metall, vorzugsweise Titan oder Edelstahl, Titan- oder Edeltahllegierungen, Karbon, Weichkunststoff, insbesondere Silikon, Hartkunststoff, insbesondere Teflon und/oder biologisch resorbierbarem Material besteht.

14. Gefäßverbinder nach einem der Ansprüche 1 bis 13, **dadurch gekennzeichnet, dass** er wenigstens einseitig mit einer Beschichtung versehen ist, vorzugsweise einer antithrombotischen Beschichtung.

15. Gefäßverbinder nach einem der Ansprüche 1 bis 14, **dadurch gekennzeichnet, dass** er T- oder Y-förmig verzweigt ist.

16. Gefäßverbinder nach einem der Ansprüche 1 bis 15, **dadurch gekennzeichnet, dass** er wenigstens an einem der äußeren Ringe (2) einen kragenförmigen Ansatz als Anschlag für einen in den Gefäßverbinder einzuführenden Applikator (20) besitzt.

17. Kit aus einem Gefäßverbinder (10) nach einem der Ansprüche 1 bis 16 und einem Applikator (20) mit dornförmigem Ansatz (22), auf welchem der Gefäßverbinder passgenau aufgesetzt werden kann, für die chirurgische Verbindung von Gefäßen und/oder Gefäßprothesen, vorzugsweise per Ligatur.
